# FASCICULE DE BREVET EUROPEEN

(11) **EP 1 811 928 B1**
(45) Date de publication et mention de la délivrance du brevet: **16.05.2012**
(21) Numéro de dépôt: 05809187.7
(22) Date de dépôt: 13.10.2005
(51) Int. Cl.: A61F 2/46

(54) **INSTRUMENTS DE POSE DE COTYLE**
INSTRUMENTE ZUM EINBRINGEN EINER HÜFTPFANNE
INSTRUMENTS FOR SETTING ACETABULAR CUP

(30) Priorité: 13.10.2004 FR 0410879
(43) Date de publication de la demande: 01.08.2007
(73) Titulaire: Gradel, Thomas, 74970 Marignier (FR)
(72) Inventeur: Gradel, Thomas, 74970 Marignier (FR)
(74) Mandataire: Poncet, Jean-François
(86) Numéro de dépôt international: PCT/FR2005/002542
(87) Numéro de publication internationale: WO 2006/040483

(56) Documents cités:
- EP-A- 0 504 521
- FR-A- 2 785 523
- FR-A- 2 809 305
- US-A- 6 152 930

## Description

### DOMAINE TECHNIQUE DE L'INVENTION

La présente invention concerne les instruments de pose d'un cotyle prothétique destiné à remplacer le cotyle naturel de la hanche.

Une prothèse totale de hanche comprend deux parties constituant une articulation à rotule, à savoir une partie femelle destinée à remplacer le cotyle naturel de la hanche et une partie mâle destinée à remplacer la tête du fémur.

La partie mâle de l'articulation comporte généralement une tige, destinée à pénétrer dans le canal médullaire du fémur, et dont l'extrémité proximale se raccorde par un col à une tête sphérique destinée à pénétrer dans le cotyle.

La partie femelle de l'articulation, qui doit remplacer le cotyle naturel de la hanche, et que l'on désignera globalement par l'appellation cotyle, comprend habituellement une cupule hémisphérique métallique à face proximale convexe et face distale concave, la face proximale convexe venant se loger dans une cavité cotyloïdienne préparée de l'os du bassin, la face distale concave recevant la face proximale convexe hémisphérique d'un insert en une matière plastique à faible coefficient de frottement tel que le polyéthylène ou une céramique, l'insert ayant lui-même une cavité distale hémisphérique coaxiale qui reçoit la tête sphérique de la partie mâle de l'articulation.

On a déjà prévu des prothèses à simple mobilité, dans lesquelles l'insert en polyéthylène ou en céramique est fixe dans la face distale concave de la cupule métallique, les mouvements de rotation de l'articulation se faisant entre la tête sphérique de la partie mâle de prothèse et la cavité distale hémisphérique de l'insert.

On a par ailleurs déjà proposé des prothèses à double mobilité, dans lesquelles l'insert en polyéthylène ou en céramique a lui-même une capacité de rotation dans la face distale concave de la cupule métallique, de sorte que les mouvements de rotation de l'articulation se font d'une part entre la face proximale convexe de l'insert et la face distale concave de la cupule métallique, et d'autre part entre la cavité distale hémisphérique de l'insert et la tête sphérique de partie mâle d'articulation.

Dans tous les cas, l'une des difficultés réside dans le positionnement et la fixation de la cupule métallique dans la cavité cotyloïdienne de l'os. La qualité de l'adaptation et de la fixation de la cupule dans la cavité cotyloïdienne est en effet essentielle pour la longévité de la prothèse, c'est-à-dire pour la durabilité de la liaison entre l'os et la cupule métallique.

Ainsi, lors de la pose de la cupule dans la cavité cotyloïdienne, il faut pouvoir utiliser un impacteur qui permet d'appliquer une force d'enfoncement de la cupule dans la cavité cotyloïdienne de l'os, qui permet d'appliquer des couples de rotation pour régler l'orientation de la cupule, et qui permet de maintenir la cupule en position fixe pendant une durée suffisante notamment pour la prise d'un ciment entre la face proximale convexe de la cupule et la cavité cotyloïdienne de l'os. Il faut ensuite pouvoir retirer l'impacteur sans exercer d'effort sur la cupule, afin de ne pas dégrader la qualité de sa fixation dans la cavité cotyloïdienne.

Mais, notamment pour les prothèses à double mobilité dans lesquelles la face distale concave de la cupule constitue une surface de glissement qui doit être parfaitement lisse, et qui est pour cela généralement polie miroir, pour un pivotement libre de l'insert dans la cupule, il faut éviter tout risque de dégradation de cette face distale concave de cupule par les instruments de pose de la cupule dans la cavité cotyloïdienne. Par exemple, un instrument radialement expansible et rétractible, qui prendrait appui en plusieurs zones discontinues de la face distale concave de cupule pour tenir la cupule au moment de la pose, conduirait nécessairement soit à une déformation permanente et localisée de la face distale concave de la cupule, soit à un maintien insuffisant, de sorte que ce type de maintien n'est pas acceptable.

Le document EP 0 504 521 A1 enseigne de prévoir un impacteur à face proximale convexe venant s'encliqueter dans la face distale concave de la cupule. Après la fixation de la cupule dans la cavité cotyloïdienne, l'impacteur est séparé de la cupule au moyen d'une barre axiale coulissante venant en appui axial au centre de la face distale convexe de glissement de la cupule et mise en mouvement au moyen d'une gâchette actionnée par le chirurgien. L'appui de la barre coulissante est de nature à dégrader localement la face de glissement de la cupule, ce qui n'est pas acceptable.

Le document FR 2 809 305 A1 enseigne de maintenir une cupule sur une tête d'extrémité d'impacteur par la création d'un vide dans un espace libre laissé entre la tête d'extrémité et la face distale concave de la cupule. Le corps d'impacteur est pour cela muni d'une pompe manuelle à piston qui permet d'aspirer l'air de l'espace libre à travers un trou traversant de la tête d'extrémité. Un joint annulaire s'oppose à la réalimentation en air de l'espace libre. L'effort de percussion est appliqué par la tête d'extrémité à la partie de face distale concave de cupule autour de l'espace libre. Le dispositif est complexe, nécessitant un assemblage de nombreuses pièces, avec plusieurs moyens d'étanchéité aux gaz, et le document ne cite pas et ne résout pas le problème des déformations de la face distale concave de cupule par suite de contraintes d'appui inégales.

On a imaginé divers autres moyens pour tenir la cupule sans prendre appui sur sa face distale concave. Un premier moyen consiste à tenir la cupule par sa lèvre circulaire, et par la partie adjacente de sa surface externe. Mais cela conduit à dégrader localement l'os dans la zone de maintien autour de la cupule, ce qui fragilise le maintien ultérieur de la cupule dans l'os.

Le document US 4,632,111 A décrit un moyen adapté à une utilisation pour la fixation d'une cupule à contre-dépouille. L'impacteur comprend une tête hémisphérique montée en bout d'un manche et venant s'engager dans la face distale concave de la cupule. Un flasque annulaire est monté coulissant sur le manche et est repoussé vers la tête par une douille vissée sur le manche. Un anneau en élastomère est pressé axialement entre la tête et le flasque, et se dilate radialement en venant ainsi porter contre la partie annulaire en contre-dépouille de la face distale concave de cupule pour la fixation de l'impacteur. Le dévissage de la douille permet le retrait du flasque, la rétraction de l'anneau en élastomère, puis le retrait de l'impacteur. Ce dispositif est complexe, constitué d'un assemblage de pièces relativement nombreuses, et inadapté à la fixation de cupules dépourvues de contre-dépouille. En outre, sa manoeuvre de vissage induit des couples parasites sur la cupule, risquant d'altérer la qualité de sa fixation dans la cavité cotyloïdienne.

Le document DE 196 28 193 A décrit une tête d'impacteur à joint annulaire élastique périphérique et à tenon de centrage s'engageant dans un trou traversant du fond de cupule. Cette structure à cupule percée n'est pas applicable à une prothèse à double mobilité.

Le document FR 2 785 523 A1 décrit un ensemble de moyens pour la pose de cotyle qui est conforme au préambule de la revendication 1. Dans cet ensemble, l'adaptateur est muni d'ailettes élastiquement déformables permettant, grâce à leur déformation élastique, un assemblage aisé de l'adaptateur et de la cupule sans risque de dégrader la cupule. L'inconvénient est que la liaison créée par l'intermédiaire des ailettes élastiques entre l'adaptateur et la cupule est trop faible pour endurer les couples d'orientation de la cupule appliqués pour l'orientation de celle-ci dans la cavité cotyloïdienne de l'os.

Ainsi, les instruments de pose de cupule actuellement connus ne permettent pas un maintien satisfaisant de la cupule pour une libre orientation et un appui suffisant de la cupule dans l'os, sans dégrader la cupule ou l'os adjacent.

Après la pose de la cupule dans la cavité cotyloïdienne, le chirurgien a généralement besoin d'adapter dans la cupule un insert d'essai, ayant sensiblement la même forme que l'insert définitif de la prothèse, et destiné à recevoir en une ou plusieurs fois des parties mâles d'articulation. Cela permet au chirurgien de tester plusieurs tailles de partie mâle d'articulation, et de faire les essais de mouvement. Le but est de permettre au chirurgien de choisir la taille la plus appropriée de partie mâle d'articulation, en fonction de la morphologie du patient. Et il enlève ensuite l'insert d'essai, pour adapter l'insert définitif dans lequel viendra se loger la tête d'articulation mâle choisie.

Pour ces opérations, en utilisant les dispositifs décrits dans les documents mentionnés ci-dessus, le chirurgien doit donc disposer d'un nombre assez important de pièces à assembler, chaque pièce devant être stérile : une cupule cotyloïdienne, un insert d'essai, un insert définitif, plusieurs parties mâles de prothèse, et des instruments complexes pour tenir la cupule pendant sa pose. En particulier, aucun des documents ci-dessus ne décrit ni ne permet de donner à la tête d'impacteur une fonction d'insert d'essai pouvant recevoir une tête fémorale.

Considérant que les patients ont des tailles diverses, le chirurgien doit nécessairement disposer d'un stock assez important de pièces, ce qui augmente le coût d'immobilisation de l'ensemble.

### EXPOSE DE L'INVENTION

Le problème proposé par la présente invention est d'éviter les inconvénients des instruments connus de pose de cotyle, en proposant une nouvelle structure d'instruments qui assure à la fois une tenue mécaniquement résistante de la cupule sur un impacteur pour la manipulation et la pose de la cupule dans une cavité cotyloidienne, et une absence totale de déformation ou de dégradation de la face distale concave de la cupule par les instruments.

Avec les moyens selon l'invention, on doit pouvoir impacter la cupule dans la cavité cotyloïdienne, l'orienter de manière précise et satisfaisante, et la fixer correctement dans la cavité cotyloïdienne, sans détériorer la surface distale concave de glissement de la cupule, et sans aucune excroissance de surface externe de la cupule ni dégradation de la zone périphérique d'os autour de la cupule.

Les moyens selon l'invention doivent pouvoir s'appliquer indifféremment à des prothèses à simple mobilité et à des prothèses à double mobilité.

Un autre but de l'invention est de réduire le nombre de pièces stériles que le chirurgien doit assembler lors de la pose d'une prothèse. On peut ainsi réduire les stocks d'instruments, et faciliter le travail du chirurgien, en réduisant les risques opératoires.

Selon un autre but de l'invention, les instruments doivent pouvoir être séparés de la cupule sans appliquer à la cupule un effort mécanique tendant à la déplacer par translation ou rotation vis-à-vis de l'os dans lequel elle est fixée, de façon à garantir une bonne qualité de fixation de la cupule dans l'os.

Pour atteindre ces buts ainsi que d'autres, l'invention propose un ensemble de moyens pour la pose de cotyle, comprenant une cupule limitée par une face proximale convexe, par une face distale concave à surface de glissement, et par une lèvre périphérique, et comprenant un adaptateur pour solidariser la cupule sur un impacteur, l'adaptateur comportant :
- des moyens de fixation, pour fixer de manière détachable l'adaptateur sur l'impacteur,
- des moyens de fixation sur la cupule, laissant un espace libre entre l'adaptateur et la cupule une fois l'adaptateur fixé à la cupule,
- un trou d'accès mettant en communication avec l'extérieur l'espace libre entre l'adaptateur et la cupule,
dans lequel :
- la cupule comprend, sur sa face distale concave, une surface annulaire de retenue, cylindrique ou légèrement conique, prolongeant la surface de glissement vers la lèvre périphérique,
- l'adaptateur comprend une surface annulaire d'engagement, cylindrique ou légèrement conique et conforme à la surface annulaire de retenue de la cupule,
- de sorte que l'adaptateur peut être retenu en force de façon étanche avec sa surface annulaire d'engagement serrée radialement dans la surface annulaire de retenue de la cupule.

Par le fait que l'adaptateur est conformé pour être retenu en force de façon étanche selon sa périphérie dans la cupule, il est en appui sur la face distale concave de cupule selon la seule surface annulaire de retenue, à l'écart de la surface de glissement, évitant de produire toute irrégularité sur la surface de glissement de la cupule.

D'autre part, grâce au fait qu'il reste un espace libre entre l'adaptateur et la cupule une fois l'adaptateur retenu en force de façon étanche selon sa surface annulaire d'engagement dans la cupule, et par le fait que l'adaptateur comporte un trou d'accès, on peut retirer ultérieurement l'adaptateur à l'écart de la cupule en injectant par le trou d'accès un fluide sous pression, avantageusement un liquide sous pression, dans l'espace libre entre l'adaptateur et la cupule. Le fluide sous pression produit une force résultante d'éjection axiale symétrique de l'adaptateur à l'écart de la cupule, et produit sur la surface de glissement de la cupule une contrainte mécanique régulièrement répartie par le fait que cette contrainte est une pression de fluide. On évite ainsi à nouveau toute déformation localisée de la surface de glissement de la cupule, et on évite toute contrainte de traction, d'oscillation ou de rotation sur la cupule.

Le blocage en force de l'adaptateur dans la cupule peut suffire pour assurer une solidarisation forte de l'adaptateur, et donc une solidarisation forte de la cupule sur l'impacteur.

On pourra toutefois préférer prévoir en outre, sur l'adaptateur, des moyens de butée destinés à venir en appui axial sur la lèvre périphérique de la cupule pour limiter la pénétration de l'adaptateur dans la cupule. De la sorte, l'impacteur peut appliquer à l'adaptateur une force d'impaction supérieure, force qui est transmise à la cupule d'une part par la surface annulaire d'engagement de l'adaptateur dans la cupule, et d'autre part par les butées en appui sur la lèvre périphérique de la cupule.

Une solidarisation encore améliorée de la cupule sur l'adaptateur peut avantageusement être obtenue en prévoyant que la surface annulaire de retenue de la cupule comprend au moins une rainure annulaire, et la surface annulaire d'engagement de l'adaptateur comprend au moins une nervure annulaire correspondante apte à s'engager dans la rainure annulaire. La profondeur des rainures et nervures sera choisie relativement faible, pour améliorer la solidarisation sans toutefois compromettre la séparation ultérieure de l'adaptateur à l'écart de la cupule.

De préférence, la cupule peut comprendre une surface de glissement sensiblement hémisphérique avantageusement polie miroir, prolongée par une surface annulaire de retenue cylindrique ou légèrement conique courte. De la sorte, l'adaptateur peut prendre appui sur la surface annulaire de retenue, qui n'est pas une surface de glissement de la cupule, de sorte que l'on réduit les contraintes mécaniques appliquées sur la surface hémisphérique de glissement.

L'adaptateur peut être solidarisé à l'impacteur par tous moyens. Toutefois, on pourra préférer des moyens de fixation de l'adaptateur sur l'impacteur comprenant un trou de fixation taraudé pratiqué dans l'adaptateur, permettant le vissage d'une portion filetée correspondante de l'impacteur. Si le trou de fixation diffère du trou d'accès, il doit être borgne.

L'adaptateur selon l'invention est utilisé comme moyen de liaison entre l'impacteur et la cupule.

Toutefois, on peut avantageusement donner à l'adaptateur une seconde fonction, à savoir une fonction d'insert provisoire d'essai, en prévoyant un adaptateur qui comprend, sur sa face distale, une cavité hémisphérique dimensionnée pour permettre l'engagement d'une tête de prothèse fémorale. La cavité hémisphérique peut être concentrique avec la face proximale convexe de l'adaptateur, en particulier pour les prothèses à double mobilité. On comprend que cela réduit le nombre de pièces à assembler par le chirurgien pour la pose de la prothèse, puisqu'il n'y a plus besoin d'un insert d'essai spécifique distinct de l'adaptateur.

Le trou d'accès à l'espace libre entre la cupule et l'adaptateur est avantageusement conformé et dimensionné pour y engager l'extrémité d'une seringue de façon étanche. De la sorte, le fluide sous pression peut être injecté par une simple seringue, instrument qui est généralement à la disposition du chirurgien, sans que cela nécessite un appareillage supplémentaire.

De préférence, le trou d'accès de l'adaptateur peut être taraudé pour remplir simultanément la fonction de trou de fixation de l'adaptateur en permettant le vissage d'une portion filetée correspondante de l'impacteur dans l'adaptateur.

Le trou d'accès de l'adaptateur peut alors avantageusement être localisé dans le fond d'une cavité hémisphérique dimensionnée pour permettre l'engagement d'une tête de prothèse fémorale.

Comme dans les prothèses connues, l'adaptateur de type insert d'essai peut être en polyéthylène. L'insert définitif peut par contre être en polyéthylène ou en céramique.

On prévoit en outre un impacteur ayant une portion filetée pour se visser dans un trou de fixation taraudé prévu sur l'adaptateur.

De préférence, l'impacteur peut comporter en outre, à la base de la portion filetée, une portion hémisphérique conformée et dimensionnée pour se loger dans une cavité hémisphérique correspondante de l'adaptateur. On répartit ainsi de manière plus régulière les contraintes mécaniques entre l'impacteur et l'adaptateur, contraintes relativement importantes qui apparaissent lors de l'impaction de la cupule dans la cavité cotyloïdienne.

Selon l'invention, on peut avantageusement concevoir de distribuer au chirurgien un adaptateur de type insert provisoire d'essai préalablement bloqué en force de façon étanche selon sa surface annulaire d'engagement dans la surface annulaire de retenue de la cupule, l'ensemble pouvant avantageusement être conditionné à l'état stérile dans une enveloppe protectrice étanche.

Lors de l'assemblage de l'adaptateur et de la cupule, assemblage qui peut être réalisé en usine de production, on peut avantageusement utiliser la succession des étapes suivantes :
a) refroidir l'adaptateur afin d'en diminuer les dimensions,
b) positionner l'adaptateur dans la cupule,
c) revenir à température ambiante pour dilater l'adaptateur une fois en place dans la cupule, l'adaptateur se trouvant ainsi bloqué en force de façon étanche selon sa surface annulaire d'engagement dans la surface annulaire de retenue de la cupule.

On prévoit en outre de préférence une étape de stérilisation par rayons gamma de l'adaptateur et de la cupule une fois solidarisés.

Après la pose de la cupule, on peut séparer l'adaptateur à l'écart de la cupule par une étape au cours de laquelle on injecte par le trou d'accès un fluide sous pression, avantageusement un liquide sous pression, dans l'espace libre entre l'adaptateur et la cupule.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue en perspective d'un ensemble selon un mode de réalisation de l'invention, comprenant une cupule cotyloïdienne dans laquelle est engagé en force un adaptateur de type insert provisoire d'impaction et d'essai ;
- la figure 2 est une vue de côté de l'ensemble de la figure 1, inséré dans une enveloppe protectrice étanche ;
- la figure 3 est une vue de face de l'ensemble de la figure 1 ;
- la figure 4 est une vue de côté en coupe diamétrale selon le plan A-A de la figure 3 ;
- la figure 5 est une vue de côté en coupe montrant un impacteur engagé dans l'insert provisoire de l'ensemble insert-cupule de la figure 4 ; et
- la figure 6 est une vue de côté en coupe illustrant une seringue engagée dans le passage de l'insert provisoire dans un ensemble insert-cupule selon l'invention.

### DESCRIPTION DES MODES DE REALISATION PREFERES

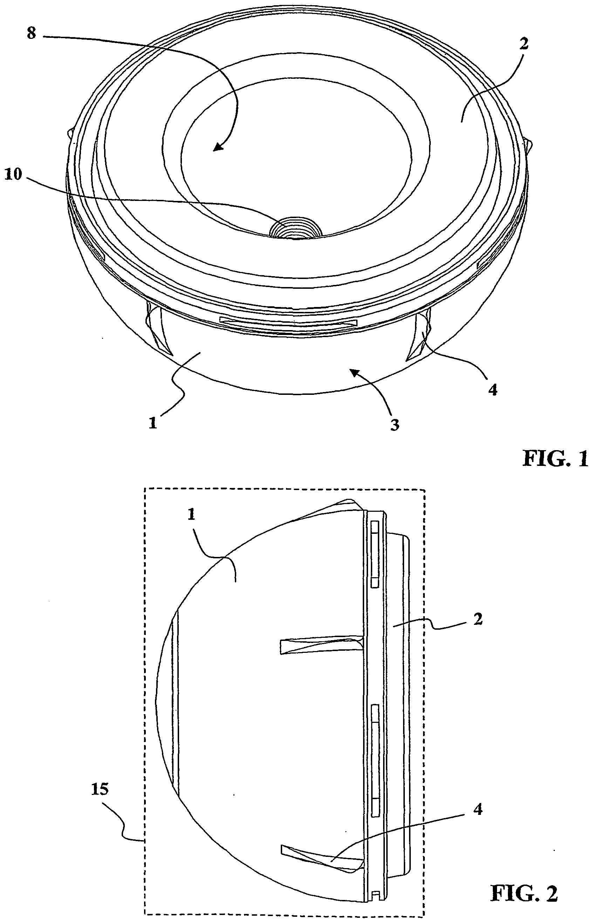

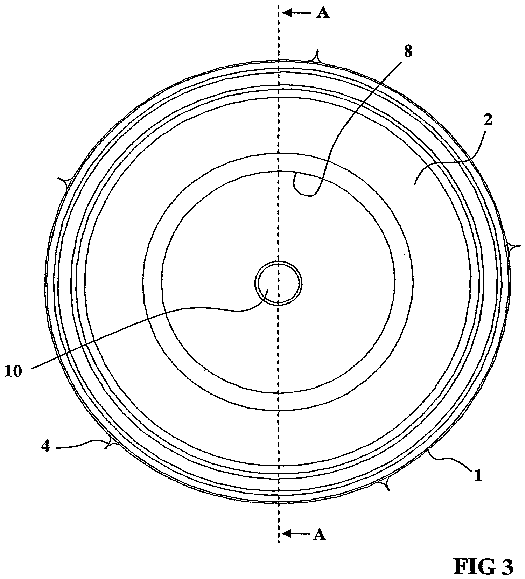

Dans le mode de réalisation illustré sur les figures 1 à 4, les instruments de pose de cotyle comprennent une cupule 1 et un adaptateur 2 conformé en insert provisoire d'impaction et d'essai.

La cupule 1 constitue la cupule définitive destinée à être fixée dans une cavité cotyloïdienne de l'os du bassin.

Par contre, l'adaptateur 2 a au moins la fonction de pièce intermédiaire entre la cupule 1 et un impacteur qui sert à manipuler la cupule 1 lors de sa pose dans la cavité cotyloïdienne, et l'adaptateur 2 est destiné à être ensuite remplacé par un insert définitif.

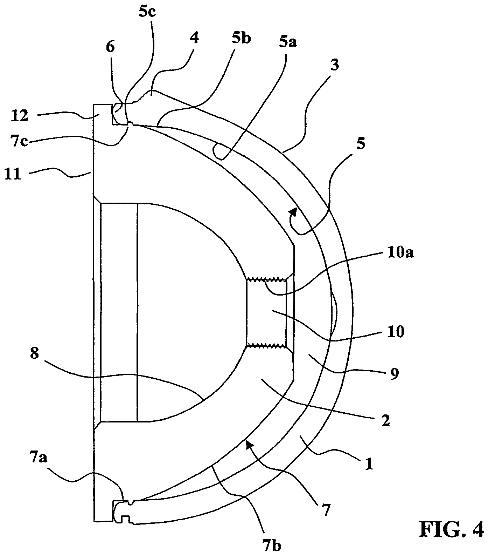

La cupule 1 est une hémisphère à paroi relativement mince, ayant une face proximale convexe 3, mieux visible sur la figure 4, de forme généralement hémisphérique avec éventuellement des ailettes d'ancrage 4, et ayant une face distale concave 5 comprenant une surface hémisphérique 5a de glissement prolongée par une surface annulaire de retenue 5b cylindrique ou légèrement conique, jusqu'à une lèvre périphérique 6.

La surface hémisphérique 5a de la cupule 1 est parfaitement lisse et régulière, de préférence polie miroir; afin de constituer une surface de glissement dans laquelle peut pivoter parfaitement un insert définitif hémisphérique ultérieurement introduit dans la cupule 1 après sa pose dans la cavité cotyloïdienne.

L'adaptateur 2 présente une forme générale arrondie limitée par une face proximale convexe 7 et une face distale 11 à cavité hémisphérique 8.

La face proximale convexe 7 de l'adaptateur 2 comprend une surface annulaire d'engagement 7a cylindrique ou légèrement conique, et une calotte centrale 7b qui reste en retrait de la forme hémisphérique constituée par la surface hémisphérique 5a de glissement de la cupule 1. De la sorte, il reste un espace libre 9 entre l'insert provisoire 2 et la cupule 1 en position emmanchée telle qu'illustrée sur la figure 4.

La surface annulaire d'engagement 7a présente une forme qui correspond à celle de la surface annulaire de retenue 5b, et un diamètre tel qu'elle est retenue en force dans la surface annulaire de retenue 5b de la cupule 1, de sorte que l'adaptateur 2 et la cupule 1 puissent constituer un sous-ensemble solidaire capable de supporter, sans mouvement relatif de l'un par rapport à l'autre, des contraintes mécaniques élevées, supérieures aux efforts qu'il faut appliquer à une cupule lors de sa pose dans la cavité cotyloïdienne.

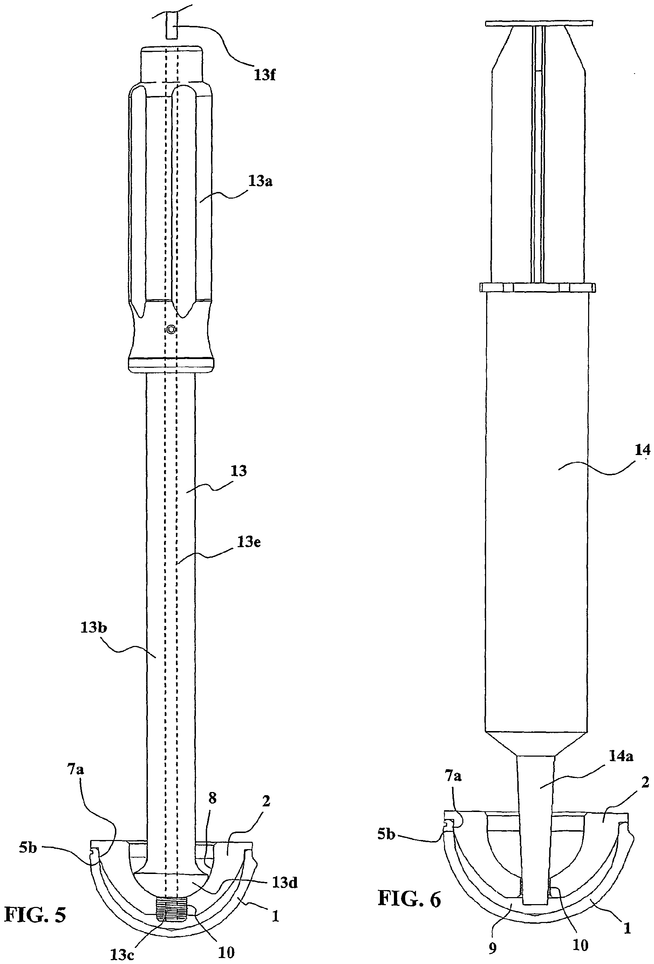

Sur les figures 5 et 6, la surface annulaire d'engagement 7a et la surface annulaire de retenue 5b sont lisses.

En alternative, dans le mode de réalisation de la figure 4, la surface annulaire de retenue 5b de la cupule 1 comprend au moins une rainure annulaire 5c, et la surface annulaire d'engagement 7a de l'adaptateur 2 comprend au moins une nervure annulaire 7c correspondante apte à s'engager dans la rainure annulaire 5c.

La cavité hémisphérique 8 de l'adaptateur 2 est dimensionnée pour recevoir une tête de rotule de partie mâle d'articulation fémorale. De la sorte, l'adaptateur 2 peut constituer un insert provisoire d'essai.

Dans le fond de l'adaptateur 2, on prévoit un trou d'accès 10 qui met en communication avec l'extérieur l'espace libre 9 entre l'adaptateur 2 et la cupule 1.

La face distale 11 de l'adaptateur 2 est bordée d'une nervure périphérique 12 circulaire qui constitue un moyen de butée venant en appui sur la lèvre périphérique 6 de la cupule 1 en position emmanchée illustrée sur la figure 4. De la sorte, la nervure périphérique 12 s'oppose à une pénétration supplémentaire de l'adaptateur 2 dans la cupule 1. Dans la réalisation illustrée sur les figures, le trou d'accès 10 constitue également un moyen de fixation détachable d'un impacteur. Il comporte pour cela un filetage intérieur 10a.

On considère maintenant la figure 5, dans laquelle on retrouve la cupule 1, l'adaptateur 2 de type insert provisoire d'essai, la cavité hémisphérique 8 de l'adaptateur 2, et le trou d'accès 10 fileté.

Sur cette figure, on a également représenté un impacteur 13, comprenant un manche 13a, une tige 13b et une extrémité filetée 13c qui vient se visser dans le trou d'accès 10 fileté. De préférence, l'impacteur 13 comporte, à la base de la portion filetée 13c, une portion hémisphérique 13d conformée et dimensionnée pour se loger dans la cavité hémisphérique correspondante 8 de l'adaptateur 2. Ainsi, l'impacteur 13 permet de manipuler la cupule 1 lors de son insertion et de son positionnement dans la cavité cotyloïdienne.

Sur la figure 6, on a illustré une étape de séparation de l'adaptateur 2 vis-à-vis de la cupule 1. Dans ce mode de réalisation, on prévoit une seringue 14 contenant un liquide approprié, par exemple du sérum physiologique, et comprenant un embout 14a que l'on vient engager de façon étanche dans le trou d'accès 10 de l'adaptateur 2. La seringue 14 permet alors d'injecter un liquide sous pression dans l'espace libre 9, provoquant la séparation de l'adaptateur 2 vis-à-vis de la cupule 1.

En alternative, pour éviter d'utiliser une seringue supplémentaire, on peut prévoir que l'impacteur 13 comprend un manche tubulaire : on a illustré ainsi sur la figure 5 un impacteur 13 comportant un canal axial 13e. Lors de l'étape de séparation, on peut introduire du liquide et une tige-piston 13f dans le canal axial 13e, ce qui force le liquide dans l'espace libre 9.

Dans le mode de réalisation illustré sur les figures, l'adaptateur 2 comporte la cavité hémisphérique 8, pour remplir la fonction d'insert provisoire d'essai. On pourra toutefois, sans sortir du cadre de l'invention, prévoir un adaptateur 2 dépourvu de cavité hémisphérique, servant alors uniquement d'adaptateur pour le positionnement de la cupule 1 dans la cavité cotyloïdienne par un impacteur 13.

Egalement, dans le mode de réalisation illustré, le trou d'accès 10 sert simultanément de moyen de fixation de l'impacteur 13 sur l'adaptateur 2. En alternative, on pourrait prévoir dans la partie centrale de l'adaptateur 2 un trou pour la fixation de l'impacteur 13, tandis que le trou d'accès 10 pour l'injection de fluide pourrait être déporté. Le trou pour la fixation de l'impacteur devrait alors être borgne.

L'utilisation des instruments selon l'invention est expliquée ci-après.

En usine, on peut assembler l'adaptateur 2 dans la cupule 1 par une procédure-comprenant les étapes :
a) on refroidit l'adaptateur 2 à une température suffisamment basse pour en diminuer légèrement les dimensions, lesdites dimensions étant initialement telles que le diamètre de la surface annulaire d'engagement 7a de l'adaptateur 2 soit légèrement plus grand que le diamètre de la surface annulaire de retenue 5b de la cupule 1 à la même température. En abaissant la température de l'adaptateur 2, on réduit son diamètre externe, afin de faciliter son engagement à l'entrée de la cupule 1 elle-même conservée à température ambiante ;
b) on positionne alors l'adaptateur 2 dans la cupule 1, jusqu'à venir en butée par la nervure périphérique 12 contre la lèvre périphérique 6 ;
c) on revient à température ambiante pour laisser dilater l'adaptateur 2 une fois en place dans la cupule 1, l'adaptateur 2 se trouvant ainsi emmanché et retenu en force de façon étanche selon sa surface annulaire d'engagement 7a dans la cupule 1.

On stérilise ensuite l'ensemble adaptateur-cupule par rayons gamma, dans une enveloppe protectrice étanche 15 (figure 2). A partir de là, l'ensemble adaptateur-cupule se trouve ainsi conditionné à l'état stérile dans l'enveloppe protectrice étanche 15.

Sur le lieu d'utilisation, c'est-à-dire dans la salle d'opération, le chirurgien retire l'enveloppe protectrice étanche 15, et peut engager un impacteur 13 stérile par vissage dans le trou d'accès 10. Le chirurgien peut ensuite manipuler la cupule 1 grâce à l'impacteur 13 et à l'adaptateur 2 emmanché, imprimant à la cupule 1 tous les efforts mécaniques nécessaires pour son positionnement et son orientation.

Le chirurgien retire ensuite l'impacteur 13 par dévissage.

Ensuite, le chirurgien peut adapter une tête de rotule dans la cavité hémisphérique 8 de l'adaptateur 2 de type insert provisoire, qui sert alors d'insert provisoire d'essai.

Une fois la partie mâle de prothèse choisie, le chirurgien peut retirer l'insert provisoire 2 à l'écart de la cupule 1 par introduction d'un liquide ou autre fluide sous pression, au moyen d'une seringue 14 comme illustré sur la figure 6, ou dans l'impacteur 13 à tige-piston 13f et à canal axial 13e comme illustré sur la figure 5.

Il adapte ensuite un insert définitif, dans la cupule 1 elle-même en place dans une cavité cotyloïdienne.

Toutes ces opérations ont été effectuées sans risque de déformation de la surface hémisphérique 8 de glissement de la cupule 1, et sans effort parasite sur la cupule 1.

La présente invention n'est pas limitée aux modes de réalisation qui ont été explicitement décrits, mais elle en inclut les diverses variantes et généralisations contenues dans le domaine des revendications ci-après.

## Revendications

1. Ensemble de moyens pour la pose de cotyle, comprenant une cupule (1) limitée par une face proximale convexe (3), par une face distale (5) concave à surface de glissement (5a), et par une lèvre périphérique (6), et comprenant un adaptateur (2) pour solidariser la cupule (1) sur un impacteur (13), l'adaptateur (2) comportant :
- des moyens de fixation (10, 10a), pour fixer de manière détachable l'adaptateur (2) sur l'impacteur (13),
- des moyens de fixation sur la cupule (1), laissant un espace libre (9) entre l'adaptateur (2) et la cupule (1) une fois l'adaptateur (2) fixé à la cupule (1),
- un trou d'accès (10) mettant en communication avec l'extérieur l'espace libre (9) entre l'adaptateur (2) et la cupule (1),
- la cupule (1) comprend, sur sa face distale (5) concave, une surface annulaire de retenue (5b), cylindrique ou légèrement conique, prolongeant la surface de glissement (5a) vers la lèvre périphérique (6),
**caractérisé en ce que** :
- l'adaptateur (2) comprend une surface annulaire d'engagement (7a), cylindrique ou légèrement conique et conforme à la surface annulaire de retenue (5b) de la cupule (1),
- de sorte que l'adaptateur (2) peut être retenu en force de façon étanche avec sa surface annulaire d'engagement (7a) serrée radialement dans la surface annulaire de retenue (5b) de la cupule (1).

2. Ensemble selon la revendication 1, **caractérisé en ce que** l'adaptateur (2) comprend des moyens de butée (12) destinés à venir en appui axial sur la lèvre périphérique (6) de la cupule (1) pour limiter la pénétration de l'adaptateur (2) dans la cupule (1).

3. Ensemble selon l'une des revendications 1 ou 2, **caractérisé en ce que** la surface de glissement (5a) de la cupule (1) est sensiblement hémisphérique, prolongée par une surface annulaire de retenue (5b) cylindrique ou légèrement conique courte.

4. Ensemble selon l'une quelconque des revendications 1 à 3, **caractérisé en ce que** la surface annulaire de retenue (5b) de la cupule (1) comprend au moins une rainure annulaire (5c), et la surface annulaire d'engagement (7a) de l'adaptateur (2) comprend au moins une nervure annulaire (7c) correspondante apte à s'engager dans la rainure annulaire (5c).

5. Ensemble selon l'une quelconque des revendications 1 à 4, **caractérisé en ce que** les moyens de fixation de l'adaptateur (2) sur l'impacteur (13) comprennent un trou de fixation (10) taraudé pratiqué dans l'adaptateur (2), permettant le vissage d'une portion filetée (13c) correspondante de l'impacteur (13).

6. Ensemble selon l'une quelconque des revendications 1 à 5, **caractérisé en ce que** le trou d'accès (10) à l'espace libre (9) entre la cupule (1) et l'adaptateur (2) est conformé et dimensionné pour y engager l'extrémité (14a) d'une seringue (14) de façon étanche.

7. Ensemble selon l'une quelconque des revendications 1 à 6, **caractérisé en ce que** le trou d'accès (10) de l'adaptateur (2) est taraudé (10a) pour remplir la fonction de trou de fixation de l'adaptateur en permettant le vissage d'une portion filetée (13c) correspondante de l'impacteur (13) dans l'adaptateur (2).

8. Ensemble selon la revendication 7, **caractérisé en ce que** l'impacteur (13) comprend un manche tubulaire à canal axial (13e) dans lequel on peut introduire un liquide et une tige-piston (13f) qui force le liquide dans l'espace libre (9).

9. Ensemble selon l'une quelconque des revendications 1 à 8, **caractérisé en ce que** l'adaptateur (2) est un insert provisoire d'essai comprenant, sur sa face distale (11), une cavité hémisphérique (8) dimensionnée pour permettre l'engagement d'une tête de prothèse fémorale.

10. Ensemble selon la revendication 9, **caractérisé en ce que** le trou d'accès (10) de l'adaptateur (2) est localisé dans le fond de la cavité hémisphérique (8).

11. Ensemble selon l'une des revendications 9 ou 10, **caractérisé en ce que** l'adaptateur (2) est en polyéthylène.

12. Ensemble selon l'une quelconque des revendications 1 à 11, **caractérisé en ce qu'**il comprend en outre un impacteur (13) ayant une portion filetée (13c) pour se visser dans un trou de fixation (10) taraudé prévu sur l'adaptateur (2).

13. Ensemble selon la revendication 12, **caractérisé en ce que** l'impacteur (13) comporte en outre, à la base de la portion filetée (13c), une portion hémisphérique (13d) conformée et dimensionnée pour se loger dans une cavité hémisphérique (8) correspondante de l'adaptateur (2).

14. Ensemble selon l'une quelconque des revendications 1 à 13, **caractérisé en ce que** l'adaptateur (2) est bloqué en force de façon étanche selon sa surface annulaire d'engagement (7a) dans la surface annulaire de retenue (5b) de la cupule (1).

15. Ensemble selon la revendication 14, **caractérisé en ce que** l'ensemble adaptateur-cupule est conditionné à l'état stérile dans une enveloppe protectrice étanche (15).

16. Procédé d'assemblage d'un adaptateur (2) et d'une cupule (1) de l'ensemble selon l'une quelconque des revendications 1 à 15, **caractérisé en ce qu'**il comprend les étapes:
a) refroidir l'adaptateur (2) afin d'en diminuer les dimensions,
b) positionner l'adaptateur (2) dans la cupule (1),
c) revenir à température ambiante pour dilater l'adaptateur (2) une fois en place dans la cupule (1), l'adaptateur (2) étant ainsi bloqué en force de façon étanche selon sa surface annulaire d'engagement (7a) dans la surface annulaire de retenue (5b) de la cupule (1).

17. Procédé d'assemblage selon la revendication 16, **caractérisé en ce qu'**il comprend une étape de stérilisation par rayons gamma de l'adaptateur (2) et de la cupule (1) une fois solidarisés.

## Claims

1. Set of acetabular cup prosthesis setting means, comprising a cup (1) delimited by a convex proximal face (3), a concave distal face (5) with a sliding surface (5a), and a peripheral lip (6), and further comprising an adapter (2) for fastening the cup (1) to an impactor (13), the adapter (2) including:
- fixing means (10, 10a) for detachably fixing the adapter (2) to the impactor (13),
- fixing means on the cup (1), leaving a free space (9) between the adapter (2) and the cup (1) once the adapter (2) is fixed to the cup (1),
- an access hole (10) through which the free space (9) between the adapter (2) and the cup (1) communicates with the outside,
- the cup (1) comprises, on its concave distal face (5), a cylindrical or slightly conical annular retaining surface (5b) extending the sliding surface (5a) toward the peripheral lip (6), **characterized in that**:
- the adapter (2) comprises a cylindrical or slightly conical annular engagement surface (7a) the same shape as the annular retaining surface (5b) of the cup (1),
- with the result that the adapter (2) may be forcibly retained in fluid-tight manner with its annular engagement surface (7a) gripped radially in the annular retaining surface (5b) of the cup (1).

2. Set according to claim 1, **characterized in that** the adapter (2) comprises abutment means (12) adapted to bear axially on the peripheral lip (6) of the cup (1) to limit the penetration of the adapter (2) into the cup (1).

3. Set according to either of claims 1 or 2, **characterized in that** the sliding surface (5a) of the cup (1) is substantially hemispherical, extended by a short cylindrical or slightly conical annular retaining surface (5b).

4. Set according to any one of claims 1 to 3, **characterized in that** the annular retaining surface (5b) of the cup (1) comprises at least one annular groove (5c) and the annular engagement surface (7a) of the adapter (2) comprises at least one corresponding annular rib (7c) adapted to be engaged in the annular groove (5c).

5. Set according to any one of claims 1 to 4, **characterized in that** the means for fixing the adapter (2) to the impactor (13) comprise a threaded fixing hole (10) in the adapter (2) for screwing in a corresponding threaded portion (13c) of the impactor (13).

6. Set according to any one of claims 1 to 5, **characterized in that** the access hole (10) to the free space (9) between the cup (1) and the adapter (2) is conformed and dimensioned for the fluid-tight engagement therein of the end (14a) of a syringe (14).

7. Set according to any one of claims 1 to 6, **characterized in that** the access hole (10) of the adapter (2) is threaded (10a) to fulfill the function of an adapter fixing hole by enabling a corresponding threaded portion (13c) of the impactor (13) to be screwed into the adapter (2).

8. Set according to claim 7, **characterized in that** the impactor (13) comprises a tubular handle with an axial passage (13e) into which may be introduced a liquid and a piston-rod (13f) that forces the liquid into the free space (9).

9. Set according to any one of claims 1 to 8, **characterized in that** the adapter (2) is a temporary trial insert comprising, on its distal face (11), a hemispherical cavity (8) dimensioned to enable the engagement of the head of a femoral prosthesis.

10. Set according to claim 9, **characterized in that** the access hole (10) of the adapter (2) is located in the bottom of the hemispherical cavity (8).

11. Set according to either of claims 9 or 10, **characterized in that** the adapter (2) is made of polyethylene.

12. Set according to any one of claims 1 to 11, **characterized in that** it further comprises an impactor (13) having a threaded portion (13c) for screwing into a threaded fixing hole (10) in the adapter (2).

13. Set according to claim 12, **characterized in that** the impactor (13) further includes, at the base of the threaded portion (13c), a hemispherical portion (13d) conformed and dimensioned to be accommodated in a corresponding hemispherical cavity (8) of the adapter (2).

14. Set according to any one of claims 1 to 13, **characterized in that** the annular engagement surface (7a) of the adapter (2) is forcibly immobilized in fluid-tight manner in the annular retaining surface (5b) of the cup (1).

15. Set according to claim 14, **characterized in that** the adapter-cup assembly is packaged in a sterile state in a sealed protective envelope (15).

16. Method of assembling an adapter (2) and a cup (1) of the set according to any one of claims 1 to 15, **characterized in that** it comprises the steps of:
a) cooling the adapter (2) to reduce its dimensions,
b) positioning the adapter (2) in the cup (1),
c) allowing the adapter (2), once in place in the cup (1), to return to room temperature to expand it so that the annular engagement surface (7a) of the adapter (2) is forcibly immobilized in fluid-tight manner in the annular retaining surface (5b) of the cup (1).

17. Assembly method according to claim 16, **characterized in that** it comprises a step of sterilizing the adapter (2) and the cup (1) by means of gamma rays once they have been fastened together.

## Patentansprüche

1. Anordnung von Mitteln für die Verlegung einer Gelenkpfanne, umfassend eine Schale (1), die durch eine proximale convexe Fläche (3), eine konkave distale Fläche (5) mit gleitfähiger Oberfläche (5a) und durch eine Umfangslippe (6) begrenzt ist, und einen Adapter (2) enthält, um die Schale (1) an einem Handhabungskörper (13) anzubringen, wobei der Adapter (2) enthält:
- Befestigungsmittel (10, 10a) zum lösbaren Befestigen des Adapters (2) an dem Handhabungskörper (13),
- wobei die Befestigungsmittel an der Schale (1) einen freien Raum (9) zwischen dem Adapter (2) und der Schale (1) lassen, wenn der Adapter (2) an der Schale (1) befestigt ist,
- wobei eine Zugangsöffnung (10) den freien Raum (9) zwischen dem Adapter (2) und der Schale (1) in Verbindung mit der Außenseite bringt,
- wobei die Schale (1) an ihrer konkaven distalen Fläche (5) eine ringförmige Haltefläche (5b) enthält, die zylindrisch oder leicht konisch ist und die gleitfähige Fläche (5a) in Richtung zur Umfangslippe (6) verlängert,
**dadurch gekennzeichnet, daß**:
- der Adapter (2) eine ringförmige Eingriffsfläche (7a) aufweist, die zylindrisch oder leicht konisch ist und mit der ringförmigen Haltefläche (5b) der Schale (1) zusammenpaßt,
- derart, daß der Adapter (2) in dichter Weise mit seiner ringförmigen Eingriffsfläche (7a) unter Kraft gehalten werden kann, indem sie radial in der ringförmigen Haltefläche (5b) der Schale (1) eingeklemmt ist.

2. Anordnung nach Anspruch 1, **dadurch gekennzeichnet, daß** der Adapter (2) Anschlagmittel (12) aufweist, die dazu bestimmt sind, an einer Umfangslippe (6) der Schale (1) in Anschlag zu kommen, zur Begrenzung des Eindringens des Adapters (2) in die Schale (1).

3. Anordnung nach einem der Ansprüche 1 oder 2, **dadurch gekennzeichnet, daß** die gleitfähige Oberfläche (5a) der Schale (1) im wesentlichen halbkugelförmig ist und durch eine ringförmige, zylindrische oder leicht konische kurze Haltefläche (5b) verlängert ist.

4. Anordnung nach irgendeinem der Ansprüche 1 bis 3, **dadurch gekennzeichnet, dass** die ringförmige Haltefläche (5b) der Schale (1) mindestens eine ringförmige Rille (5c) aufweist und die ringförmige Eingriffsfläche (7a) des Adapters (2) mindestens einen korrespondierenden Vorsprung (7c) aufweist, der geeignet ist, in die ringförmige Rille (5c) einzugreifen.

5. Anordnung nach irgendeinem der Ansprüche 1 bis 4, **dadurch gekennzeichnet, dass** die Mittel zum Befestigen des Adapters (2) an dem Handhabungskörper (13) ein Befestigungsloch (10) mit Gewinde enthalten, das in dem Adapter (2) ausgebildet ist und das. Einschrauben eines mit entsprechenden Gewinde versehenen Teiles (13c) des Handhabungskörpers (13) gestattet.

6. Anordnung nach irgendeinem der Ansprüche 1 bis 5, dadurch gekenntzeichnet, dass das Zugangsloch (10) zum freien Raum (9) zwischen der Schale (1) und dem Adapter (2) so ausgebildet und dimensioniert ist, dass dort das Ende (14a) einer Spritze (14) in dichter Weise eingreifen kann.

7. Anordnung nach irgendeinem der Ansprüche 1 bis 6, **dadurch gekennzeichnet, dass** die Zugangsöffnung (10) des Adapters (2) mit einem Gewinde (10a) versehen ist, um die Funktion des Befestigungsloches des Adapters zu erfüllen und das Einschrauben eines mit entsprechendem Gewinde versehenen Abschnittes (13c) des Handhabungskörpers (13) in den Adapter (2) zu gestatten.

8. Anordnung nach Anspruch 7, **dadurch gekennzeichnet, dass** der Handhabungskörper (13) einen rohrförmigen Stiel mit axialem Kanal (13e) aufweist, in den man eine Flüssigkeit und eine Kolbenstange (13f) einführen kann, die die Flüssigkeit in den freien Raum (9) drückt.

9. Anordnung nach irgendeinem der Ansprüche 1 bis 8, **dadurch gekennzeichnet, dass** der Adapter (2) ein provisorischer Probeeinsatz ist, der an seiner distalen Fläche (11) einen halbkugelförmigen Hohlraum (8) aufweist, der so dimensioniert ist, dass er den Eingriff eines Kopfes einer Hüftgelenksprothese gestattet.

10. Anordnung nach Anspruch 9, **dadurch gekennzeichnet, dass** die Zugangsöffnung (10) des Adapters (2) im Boden des halbkugelförmigen Hohlraumes (8) angeordnet ist.

11. Anordnung nach einem der Ansprüche 9 oder 10, **dadurch gekennzeichnet, dass** der Adapter (2) aus Polyethylen ist.

12. Anordnung nach irgendeinem der Ansprüche 1 bis 11, **dadurch gekennzeichnet, dass** sie darüber hinaus einen Handhabungskörper (13) aufweist, der einen Gewindeabschnitt (13c) hat, um sich in ein mit Gewinde versehenes Befestigungsloch (10) einzuschrauben, das an dem Adapter (2) vorgesehen ist.

13. Anordnung nach Anspruch 12, **dadurch gekennzeichnet, dass** der Handhabungskörper (13) zusätzlich an der Basis des Gewindeteiles (13c) einen halbkugelförmigen Anschnitt (13d) aufweist, der ausgebildet und dimensioniert ist, um sich in einen entsprechenden halbkugelförmigen Hohlraum (8) des Adapters (2) zu plazieren.

14. Anordnung nach irgendeinem der Ansprüche 1 bis 13, **dadurch gekennzeichnet, dass** der Adapter (2) unter Kraft in dichter Weise mit seiner ringförmigen Eingriffsfläche (7a) in der ringförmigen Haltefläche (5b) der Schale (1) blockiert ist.

15. Anordnung nach Anspruch 14, **dadurch gekennzeichnet, dass** die Gesamtheit aus Adapter-Schale in einem sterilen Zustand in einer dichten Schutzhülle (15) bereitgestellt ist.

16. Verfahren zum Zusammenbauen eines Adapters (2) und einer Schale (1) der Anordnung nach irgendeinem der Ansprüche 1 bis 15, **dadurch gekennzeichnet, dass** es folgende Schritte enthält:
a) Abkühlen des Adapters (2), um seine Abmessungen zu verringern,
b) Positionieren des Adapters (2) in der Schale (1),
c) Zurückkehren zur Umgebungstemperatur, um den Adapter (2), wenn er in der Schale (1) plaziert ist, aufzuweiten, wobei der Adapter (2) unter Kraft in dichtender Weise mit seiner ringförmigen Eingriffsfläche (7a) in der ringförmigen Haltefläche (5b) der Schale (1) blockiert ist.

17. Verfahren zum Zusammenbauen nach Anspruch 16, **dadurch gekennzeichnet, dass** es einen Schritt der Sterilisation des Adapters (2) und der Schale (1) mit Gammastrahlen enthält, wenn diese zusammengefügt sind.
